# FASCICULE DE BREVET EUROPEEN

(11) **EP 3 043 705 B1**
(45) Date de publication et mention de la délivrance du brevet: **02.10.2019**
(21) Numéro de dépôt: 14752814.5
(22) Date de dépôt: 12.08.2014
(51) Int. Cl.: A61B 5/107, A61B 5/00, G16H 40/63

(54) **PROCEDE ET SYSTEME DE CARTOGRAPHIE, PROCEDE ET SYSTEME D'EVALUATION DE L'EFFICACITE D'UNE STIMULATION MEDULLAIRE**
ABBILDUNGSVERFAHREN UND -SYSTEM SOWIE VERFAHREN UND SYSTEM ZUR BEWERTUNG DER WIRKSAMKEIT EINER MEDULLÄREN SIMULATION
MAPPING METHOD AND SYSTEM, METHOD AND SYSTEM FOR EVALUATING THE EFFICACY OF MEDULLARY SIMULATION

(30) Priorité: 13.09.2013 FR 1358850
(43) Date de publication de la demande: 20.07.2016
(73) Titulaire: Centre Hospitalier Universitaire de Poitiers, 86021 Poitiers Cedex (FR)
(72) Inventeur: RIGOARD, Philippe, F-86000 Poitiers Cedex (FR); GUETARNI, Farid, F-86300 Chauvigny (FR)
(74) Mandataire: Cabinet Camus Lebkiri
(86) Numéro de dépôt international: PCT/EP2014/067231
(87) Numéro de publication internationale: WO 2015/036191

(56) Documents cités:
- US-A1- 2001 007 950
- US-A1- 2009 005 649
- US-B1- 7 374 536
- US-B1- 8 046 241

## Description

### DOMAINE TECHNIQUE DE L'INVENTION

L'invention se rapporte au domaine médical, et notamment à la technique de stimulation médullaire, en particulier pour la prise en charge de la pathologie rachidienne post-opératoire. L'invention concerne plus particulièrement les systèmes et procédés de cartographie de douleurs dorsales ressenties par un patient, et de paresthésies ressenties suite à une stimulation médullaire visant à alléger ces douleurs.

### ARRIERE PLAN TECHNOLOGIQUE DE L'INVENTION

La technique de stimulation médullaire, aussi appelée stimulation de la moelle épinière ou neurostimulation, est utilisée pour soulager des patients souffrant de douleurs neuropathiques sévères. Cette technique consiste à émettre des impulsions électriques en direction des fibres nerveuses de la moelle épinière d'un patient, par l'intermédiaire d'au moins une électrode multi contacts. La au moins une électrode peut être d'ancienne génération (monocolonne) ou de nouvelle génération (multicolonne).

La stimulation médullaire peut être posée par voie chirurgicale ou percutanée (dans ce cas, le système nerveux central est stimulé ; la stimulation est déclenchée à partir de l'électrode dans le canal vertébral) ou bien il peut s'agir d'une stimulation nerveuse sous-cutanée (le système nerveux périphérique est alors stimulé). Pour ce faire, l'électrode a été préalablement implantée chirurgicalement sur la moelle épinière, ou dans les tissus sous-cutanés, au niveau de la zone douloureuse. Par « au niveau de la zone douloureuse », on entend :
- pour une stimulation sous-cutanée, dans une région de la zone douloureuse
- pour une stimulation médullaire, percutanée ou chirurgicale, en regard d'une certaine région de la moelle épinière dans le canal vertébral, au niveau de laquelle sont centralisées les projections afférentes d'une zone douloureuse cutanée périphérique (par exemple, une région douloureuse du membre inférieur se projette au niveau de la région médullaire, située au niveau des vertèbres thoraciques T9 à T11).

L'électrode est reliée à un boîtier de commande permettant d'activer des plots de l'électrode afin de générer un champ électrique contrôlé. Le champ électrique stimule une population neuronale, ce qui provoque une paresthésie entraînant un soulagement de la douleur.

Le choix de la ou les électrodes est crucial. En effet les résultats sont variables en fonction de l'outil thérapeutique utilisé (électrodes plus ou moins sophistiquées; électrodes percutanées ou chirurgicales, électrodes conventionnelles ou de nouvelle génération, électrodes mono colonnes ou multi colonnes, électrodes de surface de couverture plus ou moins limitée, etc). Le choix de l'outil thérapeutique est effectué en fonction de paramètres de quantification de la douleur, tels que la taille ou encore l'emplacement de la zone douloureuse. En effet, l'évaluation de ces paramètres est une étape clef permettant de choisir convenablement la ou les électrodes à implanter.

Les documents US 8,046,241 et US 7,374,536 divulguent des procédés et systèmes de cartographie de zones douloureuses.

### DESCRIPTION GENERALE DE L'INVENTION

L'invention propose un procédé d'évaluation de ces paramètres de quantification de la douleur.

L'invention est définie dans les revendications indépendantes 1 et 8 et les revendications dépendantes.

L'invention concerne donc essentiellement un procédé de cartographie de zones douloureuses, comprenant les étapes suivantes :
- Afficher une première silhouette représentant la face postérieure d'un corps, sur un premier écran
- Dessiner au moins une zone douloureuse sur ladite première silhouette affichée, indiquant la localisation d'une douleur ressentie par un patient
- Déterminer un premier nombre de pixels du premier écran, correspondant à ladite zone douloureuse
- Mesurer une distance repère entre deux repères morphologiques du patient
- Convertir ledit premier nombre de pixels en une surface cutanée douloureuse, ladite distance repère intervenant comme paramètre de ladite conversion.

La surface cutanée correspondant à la zone douloureuse dessinée par le patient est un premier paramètre de quantification de la douleur dont le procédé permet l'évaluation. Ce premier paramètre aide à choisir convenablement l'outil thérapeutique, c'est à dire l'électrode, à utiliser. En effet, si la surface cutanée est de 25 centimètres carrés, une électrode d'une couverture de 10 centimètres carrés pourra être jugée inadaptée. On note qu'avantageusement, l'écran est tactile, et le patient dessine la zone douloureuse avec ses doigts, ce qui est plus pratique que d'utiliser une souris ou un stylet.

À chaque pression tactile une délimitation par cerclage de la zone douloureuse est saisie au niveau la cartographie, plusieurs zones pouvant être dessinées avec ou sans superposition. Cette cartographie est étalonnée par rapport à la distance repère afin d'extrapoler les tracés par rapport à l'individu concerné. Le dessin est simple, rapide à réaliser et permet en plus de délimiter une surface, d'identifier une localisation et une évolution du tracé au fil du traitement de la douleur.

Outre les caractéristiques qui viennent d'être évoquées dans le paragraphe précédent, le procédé de cartographie selon l'invention peut présenter une ou plusieurs caractéristiques complémentaires parmi les suivantes, considérées individuellement ou selon toutes les combinaisons techniquement possibles.

Dans un mode de réalisation non limitatif, les repères morphologiques sont les crêtes iliaques du patient. En effet, il s'agit de points de repère invariants selon la corpulence du patient.

Dans un mode de réalisation non limitatif, le procédé de cartographie comprend l'étape suivante : corriger la surface cutanée douloureuse convertie à l'aide d'un coefficient de correction. Cette étape est basée sur le test de Bland Altman. Ce test est un test statistique permettant de gommer l'erreur intra-individuelle d'appréciation du tracé par l'opérateur (le patient ou le clinicien). Une phase de relevés de mesures avec des surfaces exactes différentes en forme et localisation ont été réalisées sur un échantillon de taille significative. Puis des comparaisons entre les surfaces dessinées et les surfaces réelles ont permis de définir un coefficient de correction (correspondant à l'écart type de la différence des moyennes observées).

Dans un mode de réalisation non limitatif, le procédé de cartographie comprend l'étape suivante : déterminer un ratio de surface cutanée douloureuse comprise dans une région de la première silhouette. Ledit ratio de surface cutanée comprise dans la région est un deuxième paramètre de quantification de la douleur dont le procédé permet l'évaluation. En effet, il est intéressant de rapporter la surface cutanée douloureuse à des notions topographiques connues de l'organisme, notamment des délimitations agréées par des organismes spécialisés telles que les délimitations Haut du dos / Bas du dos (en anglais : High Back / Low Back), ou bien des délimitations communément admises sur le plan anatomique, telles que les dermatomes. En corrélant la localisation de la surface cutanée douloureuse avec un référentiel topographique choisi, le médecin a accès à des données lui permettant de choisir convenablement ses outils thérapeutiques, c'est-à-dire les électrodes à implanter. Par exemple, une électrode peut être efficace dans une région donnée, et inefficace dans une autre. On note que la quantification de la douleur par surface topographique est importante pour déterminer la prédominance et l'intensité de certains types de douleurs et ainsi expliquer la douleur par un déterminisme lié aux racines nerveuses. Cette étape permet donc en outre de déterminer les racines nerveuses qui sont plus ou moins impliquées dans le mécanisme de douleur.

Dans un mode de réalisation non limitatif, la région correspond à un dermatome. Un dermatome est une bande de peau qui correspond à une innervation sélective d'un nerf donné de l'organisme. Grâce au procédé de cartographie selon l'invention, le médecin a accès aux pourcentages de la surface cutanée douloureuse comprise dans chaque dermatome. Dans un autre mode de réalisation non limitatif, la région correspond à une région lombaire.

Dans un mode de réalisation non limitatif, l'étape de dessin d'au moins une zone douloureuse comporte les sous-étapes suivantes :
- Dessiner d'une première manière une douleur neuropathique ;
- Dessiner d'une deuxième manière une douleur mécanique.
En effet, certains scientifiques considèrent que la stimulation médullaire n'est pas efficace sur les douleurs mécaniques. Ces sous-étapes peuvent permettre de confirmer ou d'infirmer cette considération.

L'invention concerne également un procédé d'évaluation de l'efficacité d'une stimulation médullaire, comprenant les étapes suivantes :
- Afficher une deuxième silhouette représentant la face postérieure d'un corps, sur un deuxième écran
- Dessiner au moins une zone de paresthésie sur ladite deuxième silhouette affichée, indiquant la localisation d'une paresthésie ressentie par le patient en réponse à une stimulation médullaire
- Déterminer un deuxième nombre de pixels du deuxième écran, correspondant à ladite zone de paresthésie
- Convertir ledit deuxième nombre de pixels en une surface cutanée paresthésique, la distance repère intervenant comme paramètre de ladite conversion.
- Comparer la surface cutanée paresthésique à une surface cutanée douloureuse préalablement déterminée.

On note que dans un mode de réalisation non limitatif, le deuxième écran est le premier écran évoqué précédemment. Dans un mode de réalisation non limitatif, le deuxième écran est un écran tactile et le patient dessine la zone de paresthésie avec ses doigts. Dans un mode de réalisation non limitatif, la deuxième silhouette est sensiblement identique à la première silhouette évoquée précédemment. Avantageusement, la surface douloureuse a été prédéterminée au moyen du procédé de cartographie évoqué précédemment.

En comparant la surface cutanée paresthésique et la surface cutanée douloureuse, le médecin peut déterminer si la stimulation médullaire a été efficace. En effet, la stimulation médullaire a été efficace si surface cutanée paresthésique est sensiblement égale à la surface cutanée douloureuse.

Le procédé de cartographie selon l'invention permet donc de déterminer des paramètres de quantifications de la douleur :
- de manière stricte par le calcul de la surface douloureuse, et
- de manière indirecte par l'évaluation de la surface des paresthésies et enfin par le rapport douleur/paresthésies.
Tout ceci est réalisé en fonction des zones et territoires somatopiques des fibres nerveuses.

On note les deux aspects de l'invention :
- une méthode d'évaluation d'une surface douloureuse en regard d'un dispositif médical en vue d'évaluer l'efficacité de celui-ci ;
- une méthode de diagnostic et thérapeutique (pré, per et post-opératoire) permettant de recueillir et de suivre la douleur du patient et son évolution, et permettre l'indication vers une thérapeutique de choix : on parle de stéthoscope médullaire électronique.

Outre les caractéristiques qui viennent d'être évoquées dans le paragraphe précédent, le procédé d'évaluation de l'efficacité d'une stimulation médullaire selon l'invention peut présenter une ou plusieurs caractéristiques complémentaires parmi les suivantes, considérées individuellement ou selon toutes les combinaisons techniquement possibles.

Dans un mode de réalisation non limitatif, l'étape de comparaison comporte la sous-étape suivante :
- Calculer un pourcentage de recouvrement de la surface cutanée douloureuse par la surface cutanée paresthésique.
Le médecin peut ainsi évaluer numériquement l'efficacité de la stimulation médullaire.

Dans un mode de réalisation non limitatif, le procédé d'évaluation de l'efficacité d'une stimulation médullaire comporte les étapes suivantes :
- Superposer la deuxième silhouette à une première silhouette comprenant des zones douloureuses.
Avantageusement, les zones douloureuses ont été préalablement dessinées sur la première silhouette via le procédé de cartographie évoqué précédemment. Avantageusement, la première silhouette et la deuxième silhouette sont sensiblement identiques. Il est ainsi aisé de savoir dans quelles zones douloureuses la stimulation médullaire a été efficace.

Dans un mode de réalisation non limitatif, la stimulation médullaire est réalisée lors d'une opération d'implantation de ladite électrode. Ceci est particulièrement avantageux pour implanter l'électrode à un endroit où les stimulations médullaires seront efficaces. En pratique, le patient est réveillé pendant l'opération d'implantation, et indique au médecin, grâce au procédé d'évaluation de l'efficacité d'une stimulation médullaire selon l'invention, si l'électrode est placée à un endroit approprié. Si ce n'est pas le cas, le médecin peut alors déplacer l'électrode avant de rendormir le patient et terminer l'opération d'implantation.

Dans un mode de réalisation non limitatif, la stimulation médullaire est réalisée postérieurement à une opération d'implantation de ladite électrode, par exemple quelques mois ou quelques années après l'opération. Cela permet de vérifier que le patient répond correctement aux stimulations médullaires, et que la zone douloureuse est toujours couverte par la zone de paresthésie. En effet, on observe parfois que les paresthésies s'atténuent avec le temps, voire disparaissent. Ainsi, un suivi du patient est possible.

L'invention propose également un système de cartographie, comprenant :
- Des moyens d'affichage d'une silhouette représentant la face postérieure d'un corps, sur un écran
- Des moyens de dessin d'au moins une zone sur ladite silhouette
- Des moyens de détermination d'un nombre de pixels de l'écran, correspondant à ladite zone
- Des moyens de conversion dudit nombre de pixels en une surface cutanée, au moyen d'une distance repère intervenant comme paramètre de ladite conversion.

Les zones dessinées peuvent correspondre à des zones douloureuses ou à des zones de paresthésie ressenties suite à une stimulation médullaire.

Outre les caractéristiques qui viennent d'être évoquées dans le paragraphe précédent, le système de cartographie selon l'invention peut présenter une ou plusieurs caractéristiques complémentaires parmi les suivantes, considérées individuellement ou selon toutes les combinaisons techniquement possibles.

Dans un mode de réalisation non limitatif, le système de cartographie, comprend :
- Des moyens de correction de la surface cutanée au moyen d'un critère de correction.

Dans un mode de réalisation non limitatif, le système de cartographie, comprend :
- Des moyens de détermination d'un ratio de ladite surface cutanée comprise dans une région de la silhouette.

Dans un mode de réalisation non limitatif, le système de cartographie, comprend :
- Des moyens de comparaison de la surface cutanée à une deuxième surface cutanée préalablement déterminée.

Le système de cartographie permet ainsi de comparer une surface cutanée douloureuse à une surface cutanée de paresthésie, les paresthésies étant ressenties en réponse à une stimulation médullaire.

L'invention propose également un système d'évaluation de l'efficacité d'une stimulation médullaire, comprenant :
- Le système de cartographie précédemment décrit
- Des moyens de stimulation médullaire
- Des moyens de comparaison d'une surface cutanée douloureuse à une surface cutanée paresthésique, ladite paresthésie étant ressentie en réponse à une stimulation médullaire par les moyens de stimulation médullaire.

Classiquement, les moyens de stimulation médullaire comportent au moins une électrode multi contacts reliée à un boîtier de contrôle, ladite électrode étant implantée au niveau de zones douloureuses du patient.

Dans un mode de réalisation non limitatif, le système d'évaluation de l'efficacité d'une stimulation médullaire comprend :
- Des moyens de calcul d'un pourcentage de recouvrement de la surface cutanée douloureuse par la surface cutanée paresthésique.

L'invention et ses différentes applications seront mieux comprises à la lecture de la description qui suit et à l'examen des figures qui l'accompagnent.

### BREVE DESCRIPTION DES FIGURES

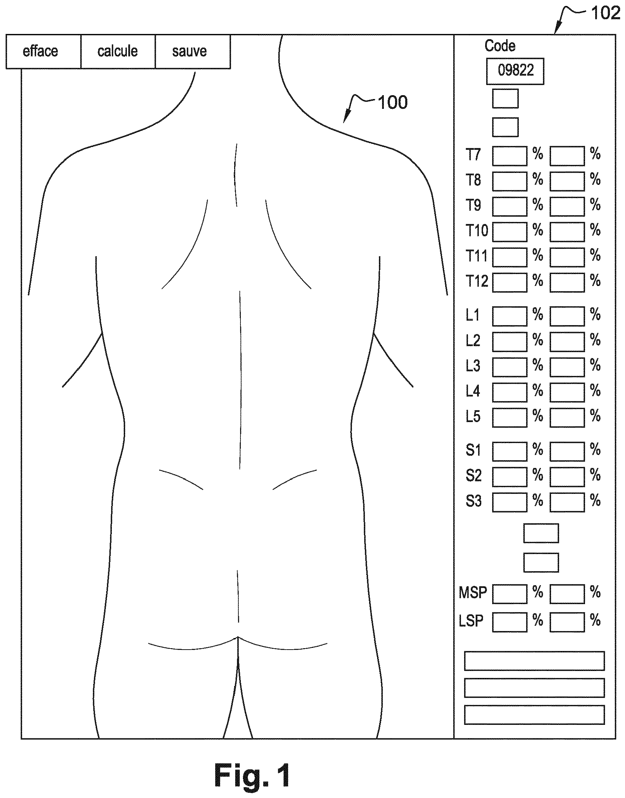

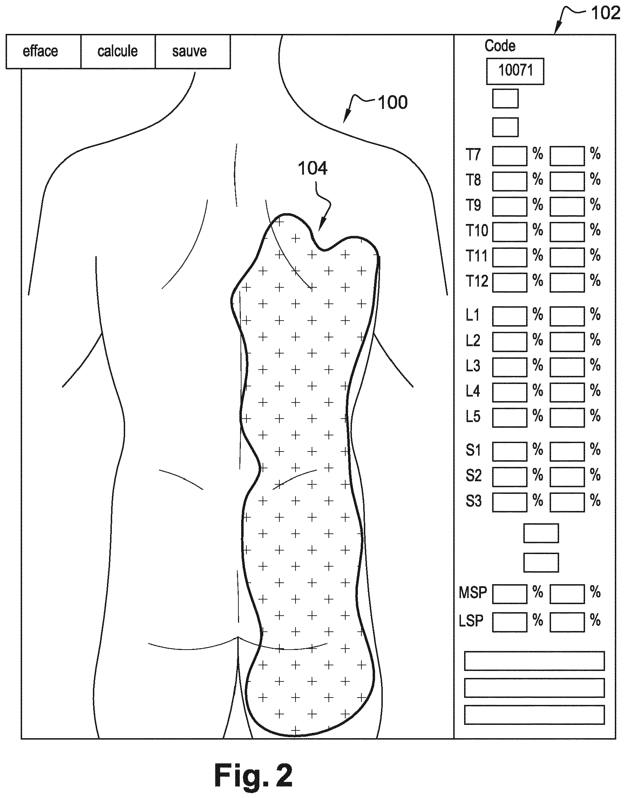

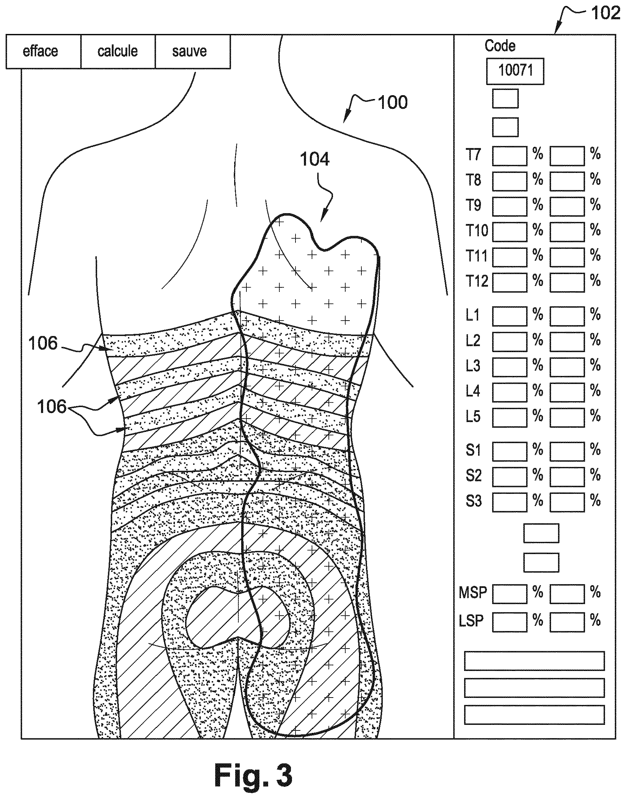

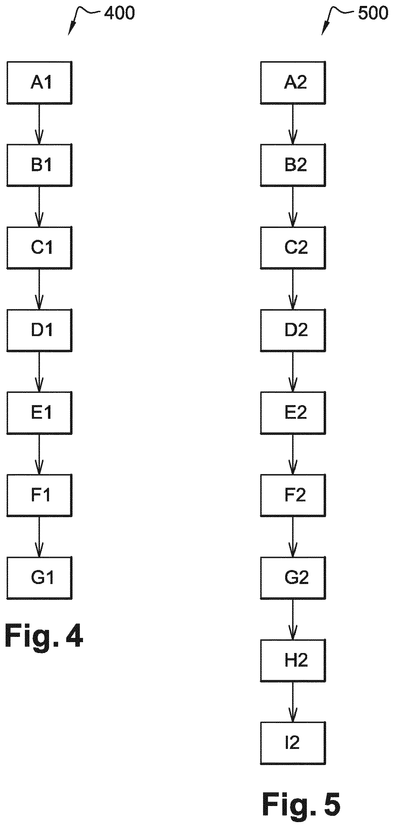

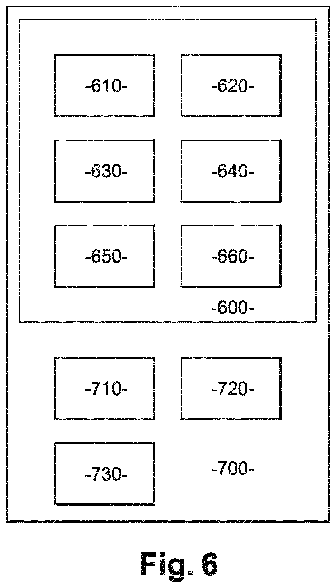

Les figures ne sont présentées qu'à titre indicatif et nullement limitatif de l'invention. Les figures montrent :
- A la figure 1, une étape d'affichage d'une silhouette sur un écran, selon un mode de réalisation du procédé de cartographie selon l'invention
- A la figure 2, une étape de dessin d'une zone douloureuse sur ladite silhouette, selon un mode de réalisation du procédé de cartographie selon l'invention
- A la figure 3, une étape d'affichage de dermatomes sur la silhouette, et de calcul de ratios de surface cutanée douloureuse correspondant à chacun de ces dermatomes
- A la figure 4, des étapes d'un mode de réalisation du procédé de cartographie selon l'invention
- A la figure 5, des étapes d'un procédé d'évaluation de l'efficacité d'une stimulation médullaire selon un mode de réalisation de l'invention
- A la figure 6, une représentation schématique d'un système de cartographie et d'un système d'évaluation de l'efficacité d'une stimulation médullaire selon un mode de réalisation de l'invention.

### DESCRIPTION DETAILLEE D'AU MOINS UN MODE DE REALISATION DE L'INVENTION

Sauf précision contraire, un même élément apparaissant sur des figures différentes présente une référence unique.

L'invention propose un procédé de cartographie de zones douloureuses, un procédé d'évaluation de l'efficacité d'une stimulation médullaire, un système de cartographie, et un système d'évaluation de l'efficacité d'une stimulation médullaire.

Le procédé de cartographie est destiné à :
- indiquer la localisation de douleurs ressenties par un patient,
- indiquer le type de ces douleurs (neuropathiques ou mécaniques) ressenties, et
- fournir des caractéristiques desdites douleurs. Ces caractéristiques sont notamment la surface cutanée concernée par la douleur, et le pourcentage de surface cutanée douloureuse comprise dans chaque dermatome ou chaque région lombaire.

Ces résultats permettent à un médecin de choisir une ou plusieurs électrodes à implanter au niveau de la ou les zones douloureuses, afin de mettre en oeuvre la technique de stimulation médullaire visant à soulager les douleurs du patient. En effet, en fonction des caractéristiques indiquées précédemment, certaines électrodes seront plus ou moins adaptées.

Plus précisément, en référence à la figure 4, le procédé de cartographie 400 comporte les étapes suivantes :
- A1 : en référence à la figure 1, afficher une première silhouette 100 représentant la face postérieure d'un corps, sur un premier écran 102. Le premier écran 102 est par exemple un écran tactile, avantageusement un écran de tablette tactile. En effet, un écran tactile permet une utilisation ergonomique, et une tablette tactile est de taille suffisamment grande pour permettre un affichage agréable, et suffisamment petite pour limiter l'encombrement. Dans le mode de réalisation décrit, la silhouette 100 est un modèle standard, non adaptée à la morphologie du patient. Cependant, un mode de réalisation dans lequel la silhouette 100 est de forme adaptée à la morphologie du patient n'est pas exclu.
- B1 : en référence à la figure 2, dessiner au moins une zone douloureuse 104 sur ladite première silhouette affichée 100, indiquant la localisation d'une douleur ressentie par le patient. Dans un mode de réalisation non limitatif dans lequel l'écran 102 est tactile, le patient délimite la zone douloureuse 104 en l'entourant avec son doigt. Dans un mode de réalisation, le patient dispose d'une palette de deux couleurs, chaque couleur représentant un type de douleur. Dans un autre mode de réalisation, la douleur est représentée d'une seule et même couleur mais des hachures permettent de différencier le type de douleur. Par type de douleur, on entend une douleur mécanique ou neuropathique. Des critères déterminés regroupés dans un questionnaire permettent de qualifier une douleur de neuropathique. Le patient peut ainsi indiquer si une zone douloureuse correspond à une douleur neuropathique ou à une douleur mécanique.
- C1 : déterminer un premier nombre de pixels du premier écran 102, correspondant à la zone douloureuse 104. On note que les nombres de pixels correspondant aux douleurs neuropathiques et mécaniques, ou à différentes zones douloureuses, s'ajoutent sauf dans le cas d'une intersection : dans ce cas, la surface commune n'est comptabilisée qu'une seule fois.

- D1 : mesurer une distance repère sur le patient. Dans le mode de réalisation décrit, la distance repère correspond à la distance entre les deux crêtes iliaques du patient. En effet, cette distance présente l'avantage d'être invariante selon la corpulence du patient. On note que d'autres repères morphologiques pourraient être utilisés, par exemple la distance entre les omoplates ou la distance entre les mastoïdes, mais la distance entre les crêtes iliaques est préférée. Il est entendu que l'étape de mesure de la distance repère peut être effectuée avant les étapes précédemment décrites, ou entre deux de ces étapes.
- E1 : convertir le premier nombre de pixels en une surface cutanée douloureuse, la distance repère intervenant comme paramètre de la conversion. La surface cutanée douloureuse est alors affichée sur le premier écran 102, ce qui permet au médecin de disposer de l'information. La surface cutanée douloureuse est par exemple exprimée en centimètres carrés.

Pour réaliser cette étape de conversion, deux coefficients sont calculés :
- un coefficient vertical égal à taille réelle du patient divisée par le nombre de pixels représentant la taille de la silhouette
- un coefficient horizontal égal à distance entre les crêtes iliaques du patient divisée par le nombre de pixels représentant la distance entre les crêtes iliaques sur la silhouette.

La surface cutanée douloureuse est alors égale au produit du coefficient horizontal, du coefficient vertical et du premier nombre de pixel correspondant à la zone douloureuse.
- F1 : déterminer un ratio de surface cutanée douloureuse comprise dans une région 106 de la première silhouette 100. En référence à la figure 3, dans un mode de réalisation, la région 106 considérée correspond à un dermatome. Dans un mode de réalisation, les régions 106 sont affichées sur la silhouette 100, superposées à la zone douloureuse 104 dessinée. La région 106 pourrait également être une région lombaire, ou toute autre région correspondant à une notion topographique connue. Avantageusement, plusieurs ratios correspondant à plusieurs régions 106 d'un référentiel topographique sont calculés. Dans un mode de réalisation, ces ratios sont exprimés en pourcentage afin d'en faciliter la lecture. Le médecin peut ainsi savoir quels dermatomes sont engagés par la douleur, et en quelles proportions. Dans un mode de réalisation, un tableau de correspondance est affiché à côté de la première silhouette 100, montrant les ratios ou les pourcentages de surface cutanée douloureuse par région considérée. La surface cutanée douloureuse est ainsi exprimée en pourcentages par rapport à la représentation surfacique des différents dermatomes de la région dorso-lombaire en particulier.
- G1 : dans un mode de réalisation, la première silhouette 100 sur laquelle sont dessinées la ou les zones douloureuses 104, ainsi que les caractéristiques de ces douleurs, sont enregistrées sur un serveur.

Le procédé de cartographie 400 peut être mis en oeuvre à différents moments dans l'espace temporel pour un patient donné. On obtient ainsi une cartographie initiale et des cartographies de suivi, ce qui permet de comparer la région douloureuse avant et après que le patient ait reçu un traitement donné et en particulier une implantation de stimulation médullaire. Les évaluations peuvent être également répétées dans le temps et à plusieurs moments de la journée ce qui permet, par implémentation de données, d'arriver à une approximation moyennée des régions douloureuses plus précise dans le temps.

Le procédé d'évaluation de l'efficacité d'une stimulation médullaire est utilisé pour :
- lors d'une opération d'implantation d'une ou plusieurs électrodes sur le patient, savoir si l'électrode est bien positionnée ou s'il est avantageux de la déplacer, et/ou
- en post-opératoire, faire un suivi du patient, c'est-à-dire vérifier que la stimulation médullaire est (encore) efficace, et si elle soulage en effet le patient sur l'intégralité des zones douloureuses.

Plus précisément, en référence à la figure 5, le procédé d'évaluation de l'efficacité d'une stimulation médullaire 500 comporte les étapes suivantes :
- A2 : réaliser les étapes du procédé de cartographie 400 précédemment décrit.
- B2 : réaliser une stimulation médullaire sur le patient à l'aide d'au moins une électrode. Dans un mode de réalisation, l'électrode a été préalablement implantée lors d'une opération chirurgicale au niveau de la zone douloureuse 104 ressentie par le patient. Dans un autre mode de réalisation, la stimulation médullaire est réalisée lors d'une opération chirurgicale destinée à implanter l'électrode.
- C2 : afficher une deuxième silhouette représentant sensiblement identique à la première silhouette 100, sur un deuxième écran. Le deuxième écran est par exemple un écran tactile, avantageusement un écran de tablette tactile. Dans un mode de réalisation non limitatif, le deuxième écran est le premier écran précédemment évoqué.
- D2 : dessiner au moins une zone de paresthésie sur la deuxième silhouette affichée, indiquant la localisation d'une paresthésie ressentie par le patient en réponse à la stimulation médullaire.
- E2 : déterminer un deuxième nombre de pixels du deuxième écran, correspondant à la zone de paresthésie.
- F2 : convertir le deuxième nombre de pixels en une surface cutanée paresthésie, la distance repère précédemment évoquée intervenant comme paramètre de la conversion. La conversion est effectuée de la même manière qu'à l'étape E1 du procédé de cartographie 400. La surface cutanée paresthésique est alors affichée sur le deuxième écran, ce qui permet au médecin de disposer de l'information.
- G2 : comparer la surface cutanée paresthésique à la surface cutanée douloureuse déterminée lors de l'étape E1 du procédé de cartographie 400.
- H2 : superposer la deuxième silhouette et la première silhouette 100 sur le deuxième écran ou sur le premier écran 102.
- I2 : dans un mode de réalisation, la deuxième silhouette sur laquelle sont dessinées la ou les zones de paresthésie, ainsi que la surface cutanée paresthésique, sont enregistrées sur le serveur.

En référence à la figure 6, le système de cartographie 600 comprend :
- Des moyens d'affichage 610 d'une silhouette représentant la face postérieure d'un corps, sur un écran
- Des moyens de dessin 620 d'au moins une zone sur ladite silhouette
- Des moyens de détermination 630 d'un nombre de pixels de l'écran, correspondant à ladite zone
- Des moyens de conversion 640 dudit nombre de pixels en une surface cutanée, au moyen d'une distance repère intervenant comme paramètre de ladite conversion.
- Des moyens de détermination 650 d'un ratio de ladite surface cutanée comprise dans une région de la silhouette
- Des moyens de comparaison 660 de la surface cutanée à une deuxième surface cutanée préalablement déterminée.

Le système de cartographie 600 permet de mettre en oeuvre le procédé de cartographie 400 selon l'invention. Les zones dessinées peuvent correspondre à des zones douloureuses ou à des zones de paresthésie ressenties suite à une stimulation médullaire.

Le système d'évaluation de l'efficacité d'une stimulation médullaire 700 comporte :
- Le système de cartographie 600
- Des moyens de stimulation médullaire 710. Classiquement, il s'agit d'au moins une électrode multi contacts implantée sous la peau du patient, reliée à un boîtier de commande.
- Des moyens de comparaison 720 d'une surface cutanée douloureuse à une surface cutanée paresthésique, ladite paresthésie étant ressentie en réponse à une stimulation médullaire par les moyens de stimulation médullaire
- Des moyens de calcul 730 d'un pourcentage de recouvrement de la surface cutanée douloureuse par la surface cutanée paresthésique.

Le système d'évaluation de l'efficacité d'une stimulation médullaire 700 permet de mettre en oeuvre le procédé d'évaluation de l'efficacité d'une stimulation médullaire 500.

## Revendications

1. Procédé de cartographie (400) de zones douloureuses, comprenant les étapes suivantes :
- Afficher (A1) une première silhouette (100) représentant la face postérieure d'un corps, sur un premier écran (102)
- Dessiner (B1) au moins une zone douloureuse (104) sur ladite première silhouette (100) affichée, indiquant la localisation d'une douleur ressentie par un patient
- Déterminer (C1) un premier nombre de pixels du premier écran (102), correspondant à ladite zone douloureuse (104)
**caractérisé par** les étapes suivantes :
- Mesurer (D1) une distance repère entre deux repères morphologiques du patient, calculer un coefficient horizontal égal à distance entre les deux repères morphologiques du patient divisée par le nombre de pixels représentant la distance entre les deux repères morphologiques sur la silhouette,
- Calculer un coefficient vertical égal à taille réelle du patient divisée par le nombre de pixels représentant la taille de la silhouette,
- Convertir (E1) ledit premier nombre de pixels en une surface cutanée douloureuse, lesdits coefficients horizontal et vertical intervenant comme paramètre de ladite conversion.

2. Procédé de cartographie (400) selon la revendication précédente, **caractérisé en ce que** les repères morphologiques sont les crêtes iliaques du patient.

3. Procédé de cartographie (400) selon l'une des revendications précédentes, **caractérisé en ce qu'**il comprend l'étape suivante :
- Corriger la surface cutanée douloureuse convertie à l'aide d'un coefficient de correction.

4. Procédé de cartographie (400) selon l'une des revendications précédentes, **caractérisé en ce qu'**il comprend l'étape suivante :
- Déterminer (F1) un ratio de surface cutanée douloureuse comprise dans une région (106) de la première silhouette (100).

5. Procédé de cartographie (400) selon l'une des revendications précédentes, **caractérisé en ce que** l'étape de dessin (B1) d'au moins une zone douloureuse (104) comporte les sous-étapes suivantes :
- Dessiner d'une première manière une douleur neuropathique
- Dessiner d'une deuxième manière une douleur mécanique.

6. Procédé d'évaluation de l'efficacité d'une stimulation médullaire (500), comprenant les étapes suivantes :
- Déterminer une surface cutanée douloureuse par le procédé de cartographie selon l'une quelconque des revendications précédentes
- Afficher (C2) une deuxième silhouette représentant la face postérieure d'un corps, sur un deuxième écran
- Dessiner (D2) au moins une zone de paresthésie sur ladite deuxième silhouette affichée, indiquant la localisation d'une paresthésie ressentie par le patient en réponse à une stimulation médullaire
- Déterminer (E2) un deuxième nombre de pixels du deuxième écran, correspondant à ladite zone de paresthésie
- Convertir (F2) ledit deuxième nombre de pixels en une surface cutanée paresthésique, la distance repère intervenant comme paramètre de ladite conversion.
- Comparer (G2) la surface cutanée paresthésique à la surface cutanée douloureuse préalablement déterminée par le procédé de cartographie selon l'une quelconque des revendications précédentes.

7. Procédé d'évaluation de l'efficacité d'une stimulation médullaire (500) selon la revendication précédente, **caractérisé en ce que** l'étape de comparaison (G2) comporte la sous-étape suivante :
- Calculer un pourcentage de recouvrement de la surface cutanée douloureuse par la surface cutanée paresthésique.

8. Système de cartographie (600), comprenant :
- Des moyens d'affichage (610) d'une silhouette (100) représentant la face postérieure d'un corps, sur un écran (102)
- Des moyens de dessin (620) d'au moins une zone (104) sur ladite silhouette (100)
- Des moyens de détermination (630) d'un nombre de pixels de l'écran (102), correspondant à ladite zone (104)
**caractérisé par** :
- Des moyens de conversion (640) dudit nombre de pixels en une surface cutanée, au moyen d'un coefficient vertical égal à une taille réelle du patient divisée par un nombre de pixels représentant une taille de la silhouette, et d'un coefficient horizontal calculé à partir d'une distance repère, selon une distance entres deux repères morphologiques du patient divisée par un nombre de pixels représentant une distance entre les deux repères morphologiques sur la silhouette, intervenant comme paramètre de ladite conversion.

9. Système de cartographie (600) selon la revendication 8, comprenant :
- Des moyens de correction de la surface cutanée convertie au moyen d'un coefficient de correction.

10. Système de cartographie (600) selon l'une des revendications 8 ou 9, comprenant :
- Des moyens de détermination d'un ratio de ladite surface cutanée comprise dans une région (106) de la silhouette (100).

11. Système de cartographie (600) selon l'une des revendications 8 à 10, comprenant :
- Des moyens de comparaison de la surface cutanée à une deuxième surface cutanée préalablement déterminée.

12. Système d'évaluation de l'efficacité d'une stimulation médullaire (700), comprenant :
- Le système de cartographie (600) selon l'une des revendications 8 à 11
- Des moyens de stimulation médullaire (710)
- Des moyens de comparaison (720) d'une surface cutanée douloureuse à une surface cutanée paresthésique, ladite paresthésie étant ressentie en réponse à une stimulation médullaire par les moyens de stimulation médullaire

13. Système d'évaluation de l'efficacité d'une stimulation médullaire (700) selon la revendication 12, comprenant :
- Des moyens de calcul (730) d'un pourcentage de recouvrement de la surface cutanée douloureuse par la surface cutanée paresthésique.

## Patentansprüche

1. Kartographieverfahren (400) von schmerzhaften Bereichen, umfassend die folgenden Schritte:
- Anzeigen (A1) einer ersten Silhouette (100) die die Rückseite eines Körpers auf einem ersten Bildschirm (102) darstellt;
- Zeichnen (B1) wenigstens eines schmerzhaften Bereichs (104) auf der genannten ersten angezeigten Silhouette (100), die die Lokalisierung eines von einem Patienten empfundenen Schmerzes anzeigt,
- Bestimmen (C1) einer ersten Anzahl von Pixeln des ersten Bildschirms (102), der dem ersten schmerzhaften Bereich (104) entspricht,
**gekennzeichnet durch** die folgenden Schritte:
- Messen (D1) eines Entfernungs-Festpunktes zwischen zwei morphologischen Festpunkten des Patienten, Berechnen eines horizontalen Koeffizienten gleich der Entfernung zwischen den zwei morphologischen Festpunkten des Patienten, die durch die Anzahl von Pixeln geteilt ist, die die Entfernung zwischen den zwei morphologischen Festpunkten auf der Silhouette darstellen,
- Berechnen eines vertikalen Koeffizienten gleich der realen Größe des Patienten, die durch die Anzahl von Pixeln geteilt ist, die die Größe der Silhouette darstellen,
- Umrechnen (F1) der genannten ersten Anzahl von Pixeln in einer schmerzhaften Haut-Oberfläche, wobei die genannten horizontalen und vertikalen Koeffizienten als Parameter der genannten Umrechnung dienen.

2. Kartographieverfahren (400) gemäß dem voranstehenden Anspruch, **dadurch gekennzeichnet, dass** die morphologischen Festpunkte die Beckenkämme des Patienten sind.

3. Kartographieverfahren (400) gemäß einem der voranstehenden Ansprüche, **dadurch gekennzeichnet, dass** es den folgenden Schritt umfasst:
- Korrigieren der schmerzhaften Hautoberfläche, die mithilfe eines Korrekturkoeffizienten umgerechnet wird.

4. Kartographieverfahren (400) gemäß einem der voranstehenden Ansprüche, **dadurch gekennzeichnet, dass** es den folgenden Schritt umfasst:
- Bestimmen (F1) eines Verhältnisses einer schmerzhaften Hautoberfläche, die in der Region (106) der ersten Silhouette (100) inbegriffen ist.

5. Kartographieverfahren (400) gemäß irgendeinem der voranstehenden Ansprüche, **dadurch gekennzeichnet, dass** der Zeichenschritt (B1) wenigstens eines schmerzhaften Bereichs (104) die folgenden Teilschritte umfasst:
- Zeichnen eines neuropathischen Schmerzes auf eine erste Weise
- Zeichnen eines mechanischen Schmerzes auf eine zweite Weise.

6. Einschätzungsverfahren der Wirksamkeit einer Rückenmarksstimulation (500), umfassend die folgenden Schritte:
- Bestimmen einer schmerzhaften Hautoberfläche durch das Kartographieverfahren gemäß irgendeinem der voranstehenden Ansprüche
- Anzeigen (C1) einer zweiten Silhouette, die die Rückseite eines Körpers darstellt, auf einem zweiten Bildschirm
- Zeichnen (D2) wenigstens eines Parästhesiebereichs auf der genannten zweiten angezeigten Silhouette, die die Lokalisierung einer Parästhesie angibt, die vom Patienten als Antwort auf eine Rückenmarksstimulation verspürt wird
- Bestimmen (E2) einer zweiten Anzahl von Pixeln des zweiten Bildschirms, die dem genannten Parästhesiebereich entspricht
- Umrechnen (F2) der zweiten Anzahl von Pixeln in einer parästhetischer Hautoberfläche, wobei die Festpunkt-Entfernung als Parameter der genannten Umrechnung dient,
- Vergleichen (G2) der parästhetischen Hautoberfläche mit der schmerzhaften Hautoberfläche, die zuvor durch das Kartographieverfahren gemäß irgendeinem der voranstehenden Ansprüche bestimmt wurde.

7. Einschätzungsverfahren der Wirksamkeit einer Rückenmarksstimulation (500) gemäß dem voranstehenden Anspruch, **dadurch gekennzeichnet, dass** der Vergleichsschritt (G2) den folgenden Teilschritt umfasst:
- Berechnen eines Abdeckungsprozentsatzes der schmerzhaften Hautoberfläche durch die parästhetische Hautoberfläche.

8. Kartographiesystem (600), umfassend:
- Anzeigemittel (610) einer Silhouette (100), die die Rückseite eines Körpers auf einem Bildschirm (102) darstellt
- Zeichenmittel (620) von wenigstens einem Bereich (104) auf der genannten Silhouette (100)
- Bestimmungsmittel (630) einer Anzahl von Pixeln des Bildschirms (102), die dem genannten Bereich (104) entspricht
**gekennzeichnet durch**:
- Umrechnungsmittel (640) der genannten Anzahl von Pixeln in einer Hautoberfläche mittels eines vertikalen Koeffizienten, der gleich einer realen Größe des Patienten geteilt durch eine Anzahl von Pixeln ist, die eine Größe der Silhouette darstellt, und eines horizontalen Koeffizienten, der ausgehend von einer Festpunkt-Entfernung gemäß einer Entfernung zwischen zwei morphologischen Festpunkten des Patienten, geteilt durch eine Anzahl von Pixeln, die eine Entfernung zwischen den zwei morphologischen Festpunkten auf der Silhouette darstellen, die als Parameter der genannten Umrechnung dienen, berechnet ist.

9. Kartographiesystem (600) gemäß Anspruch 8, umfassend:
- Korrekturmittel der Hautoberfläche, die mittels eines Korrekturkoeffizienten umgerechnet wird.

10. Kartographiesystem (600) gemäß einem der Ansprüche 8 oder 9, umfassend:
- Bestimmungsmittel eines Verhältnisses der genannten Hautoberfläche, die in einer Region (106) der Silhouette (100) inbegriffen ist.

11. Kartographiesystem (600) gemäß einem der Ansprüche 8 bis 10, umfassend:
- Vergleichsmittel der Hautoberfläche mit einer zweiten, zuvor bestimmten Hautoberfläche.

12. Einschätzungssystem der Wirksamkeit einer Rückenmarksstimulation (700), umfassend:
- Das Kartographiesystem (600) gemäß einem der Ansprüche 8 bis 11
- Mittel zur Rückenmarksstimulation (710)
- Vergleichsmittel (720) einer schmerzhaften Hautoberfläche mit einer parästhetischen Hautoberfläche, wobei die genannte Parästhesie als Antwort auf eine Rückenmarksstimulation durch die Mittel zur Rückenmarksstimulation verspürt wird.

13. Einschätzungssystem der Wirksamkeit einer Rückenmarksstimulation (700) gemäß Anspruch 12, umfassend:
- Berechnungsmittel (730) eines Abdeckungsprozentsatzes der schmerzhaften Hautoberfläche durch die parästhetische Hautoberfläche.

## Claims

1. Method for mapping (400) painful zones, including the following steps:
- Displaying (A1) a first silhouette (100) representing the rear face of a body, on a first screen (102)
- Drawing (B1) at least one painful zone (104) on said first displayed silhouette (100), indicating the location of a pain felt by a patient
- Determining (C1) a first number of pixels on the first screen (102), corresponding to said painful zone (104),
**characterized in that**:
- Measuring (D1) a reference distance between two morphological reference points on the patient, calculate a horizontal coefficient equal to the distance between the two morphological reference points on the patient divided by the number of pixels representing the distance between the two morphological reference points on the silhouette,
- Calculate a vertical coefficient equal to the actual size of the patient divided by the number of pixels representing the size of the silhouette,
- Converting (E1) said first number of pixels into a painful cutaneous surface, said horizontal and vertical coefficient being used as a parameter in said conversion.

2. Method for mapping (400) according to the preceding claim, **characterised in that** the morphological reference points are the iliac crests of the patient.

3. Method for mapping (400) according to one of the preceding claims, **characterised in that** it includes the following step:
- Correcting the converted painful cutaneous surface with the aid of a correction coefficient.

4. Method for mapping (400) according to one of the preceding claims, **characterised in that** it includes the following step:
- Determining (F1) a ratio of painful cutaneous surface comprised in a region (106) of the first silhouette (100).

5. Method for mapping (400) according to one of the preceding claims, **characterized in that** the step of drawing (B1) at least one painful zone (104) comprises the following sub-steps:
- Drawing in a first manner a neuropathic pain
- Drawing in a second manner a mechanical pain.

6. Method for evaluating the efficacy of medullary stimulation (500), including the following steps:
- Determining a painful cutaneous surface by the method for mapping according to one of the preceding claims,
- Displaying (C2) a second silhouette representing the rear face of a body, on a second screen
- Drawing (D2) at least one paraesthesia zone on said second displayed silhouette, indicating the location of a paraesthesia felt by the patient in response to medullary stimulation
- Determining (E2) a second number of pixels on the second screen, corresponding to said paraesthesia zone
- Converting (F2) said second number of pixels into a paraesthesic cutaneous surface, the reference distance being used as a parameter in said conversion,
- Comparing (G2) the paraesthesic cutaneous surface with the painful cutaneous surface determined beforehand by the mapping method according to any of the preceding claims.

7. Method for evaluating the efficacy of medullary stimulation (500) according to the preceding claim, **characterised in that** the comparison step (G2) comprises the following sub-step:
- Calculating a percentage coverage of the painful cutaneous surface by the paraesthesic cutaneous surface.

8. Mapping system (600), including:
- Means for displaying (610) a silhouette (100) representing the rear face of a body, on a screen (102)
- Means for drawing (620) at least one zone (104) on said silhouette (100)
- Means for determining (630) a number of pixels on the screen (102), corresponding to said zone (104),
**characterized by** :
- Means for converting (640) said number of pixels into a cutaneous surface, by means of a vertical coefficient equal to the actual size of the patient divided by the number of pixels representing the size of the silhouette, and
a horizontal coefficient calculated from a distance between two morphological reference points on the patient divided by the number of pixels representing the distance between the two morphological reference points on the silhouette of being used as a parameter in said conversion.

9. Mapping system (600) according to claim 8, including:
- Means for correcting the converted cutaneous surface by means of a correction coefficient.

10. Mapping system (600) according to one of claims 8 or 9, including:
- Means for determining a ratio of said cutaneous surface comprised in a region (106) of the silhouette (100).

11. Mapping system (600) according to one of claims 8 to 10, including:
- Means for comparing the cutaneous surface with a second cutaneous surface determined beforehand.

12. System for evaluating the efficacy of medullary stimulation (700), including:
- The mapping system (600) according to one of claims 8 to 11
- Medullary stimulation means (710)
- Means for comparing (720) a painful cutaneous surface with a paraesthesic cutaneous surface, said paraesthesia being felt in response to medullary stimulation by the medullary stimulation means.

13. System for evaluating the efficacy of medullary stimulation (700) according to claim 12, including:
- Means for calculating (730) a percentage coverage of the painful cutaneous surface by the paraesthesic cutaneous surface.
